# EUROPÄISCHE PATENTSCHRIFT

(11) **EP 4 337 120 B1**
(45) Veröffentlichungstag und Bekanntmachung des Hinweises auf die Patenterteilung: **14.08.2024**
(21) Anmeldenummer: 22728201.9
(22) Anmeldetag: 09.05.2022
(51) Int. Cl.: A61B 17/72

(54) **MARKNAGEL ZUR TRANSVERSALEN DISTRAKTION**
INTRAMEDULLARY NAIL FOR TRANSVERSAL DISTRACTION
CLOU CENTROMÉDULLAIRE POUR EXTENSION TRANSVERSALE

(30) Priorität: 12.05.2021 DE 102021112429
(43) Veröffentlichungstag der Anmeldung: 20.03.2024
(73) Patentinhaber: Stauch, Roman, 97959 Assamstadt (DE)
(72) Erfinder: Stauch, Roman, 97959 Assamstadt (DE)
(74) Vertreter: Caspary, Karsten
(86) Internationale Anmeldenummer: PCT/EP2022/062440
(87) Internationale Veröffentlichungsnummer: WO 2022/238303

(56) Entgegenhaltungen:
- FR-A1- 2 653 006
- US-A1- 2014 222 094
- US-A1- 2015 133 937
- US-A1- 2017 143 387

## Beschreibung

### Gebiet der Erfindung

Die vorliegende Erfindung betrifft einen intramedullären medizinischen Marknagel zur transversalen Distraktion eines Röhrenknochens, insbesondere einen voll implantierbaren aktiven medizinischen Marknagel.

### Stand der Technik

Bei einer Vielzahl von Patienten mit einer diabetischen Vorerkrankung besteht das Problem der unzureichenden Durchblutung der Extremitäten, vor allem im Bereich der Unterschenkel. In kritischen Fällen kann ein negativer Krankheitsverlauf zu einer Amputation führen. Die Orthopädie hat auf dem Gebiet der Extremitätenkorrektur hierzu verschiedene Ansätze entwickelt, unter anderem wurde in einigen Fällen festgestellt, dass durch eine Querdistraktion bzw. transversale Distraktion eines Röhrenknochens die Durchblutung der entsprechenden Extremität deutlich verbessert und dadurch eine Amputation verhindert werden kann. In der medizinischen Fachliteratur ist dieser Zusammenhang beispielsweise in Chen, Y. et al., Proximal Tibial Cortex Transverse Distraction Facilitating Healing and Limb Salvage in Severe and Recalcitrant Diabetic Foot Ulcers, Clin Orthop Relat Res (2020), 478:836-851 beschrieben.

In der Orthopädie sind hierzu Vorrichtungen bekannt, externe Fixateure genannt, die beispielsweise bei Behandlungen verwendet werden, bei denen ein Teil des Unterschenkelknochens herausgetrennt und durch transversale Distraktion neues Knochengewebe generiert und dabei die Durchblutung des umgebenden Gewebes deutlich verbessert wird. Vor allem durch die lange Tragedauer des extern am Unterschenkel angebrachten Fixateurs ist das Risiko einer Infektion stark erhöht. Darüber hinaus ist die Bewegung und der Tragekomfort des Patienten stark eingeschränkt, es besteht die Gefahr, dass der Fixateur durch äußere Stoß- oder Druckkräfte aus seiner vorbestimmten Stellung gebracht wird, was den Heilungsprozess negativ beeinflusst, der Aufwand für die Pflege des Fixateurs ist hoch und es drohen Infektionen über die Elemente (Pins), die sowohl innerhalb als auch außerhalb des Körpers angeordnet sind.

Marknägel zur axialen Distraktion eines Röhrenknochens sind unter anderem aus der EP 2 990 002 A1 bekannt. Die US 2002/0165544 A1 und die US 2017/0143387 A1 offenbaren einen Marknagel, der an seinen Enden mittels eines Ausdehnungsmechanismus an einem Röhrenknochen fixiert wird. Der Ausdehnungsmechanismus umfasst dabei auch eine eng begrenzte radiale Ausdehnung. In der US 2002/0165544 A1 sind zu diesem Zweck mit axialen Schlitzen versehene Hülsen außen auf dem Kern des Marknagels angeordnet, die mittels eines axialen Schraubmechanismus derart in ihrer Längsrichtung gequetscht werden können und sich damit ähnlich einem Faltenbalg teilweise radial erstrecken und eine Klemmung an den Knochenenden bewirken können. In der US 2017/0143387 A1 wird die Befestigung des Marknagels durch zwei radial ausfahrbare Fixierelemente bewirkt, die durch eine Verschiebung gegeneinander von der äußeren Oberfläche des Marknagels herausfahren und dadurch gleichmäßig auf beiden Seiten eine radiale Erweiterung erreichen. Der Umfang der radialen Erweiterung der Fixierelemente in der US 2017/0143387 A1 ist durch den Durchmesser des Marknagels beschränkt.

Marknägel mit einem Ausdehnungsmechanismus sind außerdem aus der US 2015/0133937 A1 bekannt, wobei der Ausdehnungsmechanismus hierbei verwendet wird, um den Marknagel mit dem Knochen zu fixieren.

Eine medizinische Vorrichtung für eine andere operative Verwendung, die dazu dient, einen fehlenden Röhrenknochen mittels Kallusdistraktion zu rekonstruieren und die dafür eine Expansionsvorrichtung zur Knochenexpansion aufweist, ist in der WO 2013/023898 A1 offenbart. Die Expansionsvorrichtung weist dabei in Längsrichtung hintereinander eine Mehrzahl von Ausdehnungseinrichtungen auf, die als Kammern ausgebildet sind, die durch eine Pumpe mit einem Medium zur Ausdehnung befüllt werden.

Nachteilig an den Vorrichtungen des Standes der Technik ist insbesondere, dass sie keine zuverlässige und möglichst schonende technische Lösung für die transversale Distraktion bereitstellen. Bei einigen bekannten Vorrichtungen ist der Umfang der Querdistraktion im Wesentlichen auf den Durchmesser des Marknagels beschränkt. Einige andere lassen sich nicht ausreichend genau einstellen oder müssen intraoperativ oder häufig invasiv eingestellt werden.

### Aufgabe der Erfindung

Es ist daher die Aufgabe der vorliegenden Erfindung, einen Marknagel bereitzustellen, wobei aus dem Stand der Technik bekannte Systeme verbessert werden sollen. Insbesondere sollen die Nachteile des Standes der Technik gelindert oder behoben werden. Angestrebt wird ein Marknagel, der eine einfache Konstruktion aufweist und eine zuverlässige transversale Distraktion mittels minimalinvasivem Eingriff ermöglicht.

Diese Aufgabe wird durch den Gegenstand des Anspruchs 1 gelöst. Vorteilhafte Ausführungsformen sind Gegenstand der abhängigen Ansprüche.

### Zusammenfassung der Erfindung

Ein Aspekt der Erfindung betrifft einen Marknagel zur transversalen Distraktion eines Röhrenknochens, wobei der Marknagel einen sich in einer axialen Richtung des Marknagels erstreckenden, zumindest teilweise hohlen Rohrkörper, mindestens ein Verriegelungsmittel zum Verriegeln des Rohrkörpers in einem Endabschnitt des Röhrenknochens, ein erstes Innenteil und ein zweites Innenteil, die jeweils innerhalb des Rohrkörpers in axialer Richtung gegeneinander oder miteinander verschiebbar angeordnet sind, einen Antrieb innerhalb des Rohrkörpers zum axialen Verschieben des ersten Innenteils relativ zu dem zweiten Innenteil, mindestens eine Längsausnehmung des Rohrkörpers und mindestens einen Hebelmechanismus umfasst, der mindestens ein Hebelelement umfasst, das dazu eingerichtet ist, in radialer Richtung aus dem Rohrkörper ausgefahren und wieder eingefahren zu werden, wobei die Verschiebung des ersten Innenteils bezüglich des zweiten Innenteils ein Herausbewegen bzw. Ausfahren des mindestens einen Hebelelements aus der mindestens einen seitlichen Längsausnehmung des Rohrkörpers oder ein Hineinbewegen bzw. Einfahren des mindestens einen Hebelelements in die mindestens eine seitliche Längsausnehmung des Rohrkörpers bewirkt.

Der Umfang der transversalen Distraktion ist damit wesentlich größer als bei bisherigen Marknägeln und nicht auf den Durchmesser des Marknagels beschränkt. Es wird ein inkrementelles Ausfahren und Einfahren des Hebelmechanismus zur transversalen Distraktion ermöglicht, wobei keine externen Elemente am Körper des Patienten aufgesetzt werden müssen. Die Verschiebung der Innenteile relativ zueinander mittels Antrieb kann bevorzugt durch Druck oder Zug erfolgen. Zudem ist die Einstellung des Umfangs der transversalen Distraktion mit dem erfindungsgemäßen Marknagel ohne Eingriff durch berührungslose Energieübertragung möglich. Der gesamte Vorgang der transversalen Distraktion erfolgt minimalinvasiv. Damit wird ein aktiver, voll implantierbarer Marknagel für die transversale Distraktion bereitgestellt, wobei die Implantation und Explantation minimalinvasiv erfolgen kann.

In Ausführungsformen kann das mindestens eine Hebelelement in einer eingefahrenen Stellung axial mit der Längsachse des Marknagels ausgerichtet sein und in einer ausgefahrenen Stellung um mindestens 30°, bevorzugt um mindestens 45°, noch bevorzugter um mindestens 60° und am meisten bevorzugt um mindestens 80° bezüglich der Längsachse des Marknagels ausgerichtet sein. Dies ermöglicht ein stufenloses Einstellen der transversalen Distraktion, die der Patient gegebenenfalls selbst vornehmen kann. Mehrere Hebelmechanismen im Marknagel, z. B. zwei bis vier, sorgen für eine ausreichende radiale Ausdehnung und damit für eine zuverlässige und gleichmäßige transversale Distraktion in den betroffenen Bereichen.

In bevorzugten Ausführungsformen kann der Antrieb axial am Rohrkörper abgestützt sein. Dadurch kann eine zuverlässige Verschiebung des ersten Innenteils bezüglich des zweiten Innenteils erfolgen.

Bei vorteilhaften Ausführungsformen kann das mindestens eine Hebelelement dazu eingerichtet sein, bei Bewegung des Marknagels in Längsrichtung ohne Betätigung des Antriebs eingefahren zu werden. Demgemäß kann, falls der Antrieb ausfallen sollte oder die Rückstellung nicht vom Antrieb vorgenommen werden kann, der Marknagel dennoch möglichst schonend entfernt werden, weil eine Verschiebung der beiden Innenteile relativ zueinander ohne Antrieb möglich ist. Diese Gewährleistung einer optimalen Ausfallsicherheit stellt einen erheblichen Vorteil gegenüber bisherigen Vorrichtungen dar.

In einer weiteren vorteilhaften Ausführungsform kann das zweite Innenteil integral mit dem Rohrkörper ausgebildet sein. Dies ermöglicht eine Konstruktion mit weniger Komponenten. Das Einfahren und Einklappen des Hebelmechanismus kann dann mit dem Antrieb erfolgen.

Bei bevorzugten Ausführungsformen kann der Antrieb als ein elektrischer, magnetischer, elektro-magnetischer, hydraulischer, formgedächtnisbasierter, piezoelektrischer oder pneumatischer Antrieb ausgebildet sein. Insbesondere ermöglichen derartige Arten von Antrieben eine Verstellung der transversalen Distraktion ohne invasiven Eingriff und/oder ohne Körperkontakt. Darüber hinaus sind beispielsweise mit einem elektromechanischen Linearantrieb sehr kleine Verstellschritte möglich, was für die Kallusdistraktion und den medizinischen Heilprozess sehr vorteilhaft ist. Die oben genannten Antriebsformen sind günstig, zuverlässig und ermöglichen einen einfachen Aufbau.

In einer vorteilhaften Ausführungsform kann der Antrieb einen Motor, ein Getriebe und vorzugsweise ein Spindelelement aufweisen. Eine solche Konstruktion ermöglicht ebenfalls extrem kleine Verstellschritte und bietet einen zuverlässigen, einfachen Aufbau des Marknagels.

Bei typischen Ausführungsformen können das erste Innenteil und das zweite Innenteil eine Halbzylinderform aufweisen. Dadurch entsteht ein einfacher Aufbau, dessen Bestandteile auf unproblematische Weise hergestellt werden können. Ebenfalls ist es möglich, dass das erste Innenteil und das zweite Innenteil andere komplementäre Formen bzw. Querschnitte aufweisen. In einem weiteren Ausführungsbeispiel kann der Marknagel einen im Wesentlichen polygonalen Querschnitt aufweisen, damit können das erste Innenteil und das zweite Innenteil entsprechende polygonal passende Querschnitte bzw. Teilquerschnitte aufweisen, damit eine relative Verschiebung der beiden Innenteile zueinander erfolgen kann. Andere Querschnittsformen sind möglich. Es ist nicht zwingend erforderlich, dass die Querschnittsfläche des ersten Innenteils gleich der Querschnittsfläche des zweiten Innenteils ist.

In besonderen Ausführungsformen kann das mindestens eine Hebelelement als Kipphebel ausgebildet sein, der ein Langloch und eine Gelenkeinrichtung aufweist, wobei die Gelenkeinrichtung drehbar an und axial festgelegt mit dem ersten Innenteil verbunden ist und wobei das zweite Innenteil ein Führungselement aufweist, das in dem Langloch des Kipphebels geführt ist. Damit ist der Hebelmechanismus vergleichsweise einfach konstruiert, da der Kipphebel lediglich ein Langloch sowie eine Gelenkeinrichtung aufweisen muss, wobei die Gelenkeinrichtung ein an einem Ende des Kipphebels angeordneter Drehbolzen sein kann und ein Führungsbolzen in dem Langloch zwangsgeführt ist. Ein Kipphebel kann gerade sein, abgerundete Kanten aufweisen und eine konisch zulaufende Form haben. Andere Ausführungsformen können gekrümmte oder unregelmäßig geformte Kipphebel aufweisen. In weiteren Ausführungsformen ist auch eine Umkehrung des für Kipphebel beschriebenen Funktionsprinzip möglich, d. h. dass beispielsweise die Führung des Kipphebels durch eine Nut oder eine Ausnehmung im ersten oder zweiten Innenteil vorgegeben ist. Auf diese Weise lässt sich auch eine Ausführungsform mit zwei Kipphebeln bilden, bei der die Kipphebel die gleiche Form aufweisen und sich synchron geführt bei der Verschiebung der Innenteile relativ zueinander nach Art eines Parallelogramms verstellen.

Weitere typische Ausführungsformen umfassen einen Hebelmechanismus, wobei das mindestens eine Hebelelement als Kniehebel ausgebildet sein kann. Die Führung des Kniehebels kann damit ohne Langloch und ohne Führungsbolzen erfolgen.

Bei weiteren typischen Ausführungsformen kann das mindestens eine Hebelelement als eine Mehrzahl von gelenkig miteinander verbundenen Kettengliedern ausgebildet sein, wobei ein Kettenglied der Mehrzahl von Kettengliedern axial an das erste Innenteil angrenzt und ein anderes der Mehrzahl von Kettengliedern an das zweite Innenteil angrenzt, wobei die Verschiebung des ersten Innenteils relativ zu dem zweiten Innenteil ein Auffalten und Hervortreten von mindestens zwei Kettengliedern durch eine seitliche Längsausnehmung des Rohrkörpers bewirkt. Damit ist eine radiale Distraktion durch mindestens zwei Kettenglieder möglich, aber auch durch mehr als zwei Kettenglieder, die in ausgefahrener Stellung nicht mehr axial ausgerichtet sind. Die Konstruktion mit mehreren Kettengliedern stellt ebenfalls einen stabilen und einfachen Aufbau dar. Die Führung der Kettenglieder kann dabei so eingerichtet sein, dass das Ende eines Kettenglieds in Radialrichtung weiter von dem Rohrkörper vorsteht als das Ende eines anderen Kettenglieds. Es versteht sich, dass die Kettenglieder nicht alle gleich lang sein müssen. Ebenso ist es möglich, dass die Kettenglieder eine gebogene oder unregelmäßige Form aufweisen, d. h. sie müssen nicht notwendigerweise gerade geformt sein. Auch Kombinationen von geraden und unregelmäßig geformten Kettengliedern sind möglich. Unter dem Begriff Kettenglieder sollen auch flexible Bänder oder Schubketten mit mehreren Segmenten verstanden werden. Entscheidend für die Eignung sind Elemente, die im weitesten Sinne die Funktion von Schwenkachsen beim Zusammenschieben eines Verbundes von Segmenten ausüben.

In bevorzugten Ausführungsformen können die Hebelelemente bzw. Kettenglieder auf der vom Rohrkörper nach außen gerichteten Seite entsprechend gestaltete Oberflächen aufweisen, die die Wirkung der radialen Distraktion verstärken. Der Marknagel an sich und seine Bestandteile können typischerweise aus biokompatiblem Material ausgebildet sein, wie zum Beispiel Implantatstahl, andere geeignete Metalllegierungen, geeignete Kunststoffe und dergleichen. Zu den geeigneten Herstellungsverfahren gehört beispielsweise auch der 3D-Druck.

Typische Ausführungsformen können derart ausgestaltet sein, dass an den freien, nach au-ßen gerichteten Enden der Kipphebel bzw. Hebelelemente eine oder mehrere Platten gelenkig angeordnet sein können, die im eingefahrenen Zustand des Hebelmechanismus die Längsausnehmung(en) abdeckt bzw. abdecken und im ausgefahrenen Zustand den radialen Druck des Hebelmechanismus flächig verteilen. Beispielsweise ist eine Ausführungsform mit zwei Kipphebeln denkbar, an deren Ende jeweils gelenkig eine gemeinsame längliche Platte befestigt ist, die im eingefahrenen Zustand eine Längsausnehmung des Rohrkörpers abdeckt und im ausgefahrenen Zustand vollständig radial nach außen bewegt wird, vorzugsweise parallel zur Außenfläche des Rohrkörpers.

### Kurze Beschreibung der Figuren

Nachfolgend werden bevorzugte Ausführungsformen anhand der beigefügten Figuren näher beschrieben, in denen:
- Fig. 1: eine schematische Querschnittsansicht einer bevorzugten Ausführungsform des erfindungsgemäßen Marknagels in einer ersten Stellung ist;
- Fig. 2: eine schematische Querschnittansicht einer weiteren Ausführungsform des erfindungsgemäßen Marknagels in einer ersten Stellung ist;
- Fig. 3: eine perspektivische Ansicht einer weiteren Ausführungsform des erfindungsgemäßen Marknagels in einer zweiten Stellung ist;
- Fig. 4: eine schematische Querschnittansicht des Marknagels aus Fig. 1 in der ersten Stellung in einem Röhrenknochen eingesetzt ist;
- Fig. 5: eine schematische Querschnittansicht des Marknagels aus Fig. 1 in der zweiten Stellung in einem Röhrenknochen eingesetzt ist;
- Fig. 6: eine schematische Querschnittansicht des Marknagels aus Fig. 1 nach Verlassen der zweiten Stellung ist;
- Fig. 7: eine schematische Querschnittansicht des Marknagels aus Fig. 1 in einer herausgezogenen Stellung ist;
- Fig. 8: eine schematische Darstellung eines Hebelmechanismus gemäß einer weiteren Ausführungsform in einer ersten Stellung ist; und
- Fig. 9: eine schematische Darstellung eines Hebelmechanismus aus Fig. 8 in einer zweiten Stellung ist.

Ausführliche Beschreibung von Ausführungsbeispielen

In der folgenden Beschreibung werden anhand der Figuren Ausführungsbeispiel erläutert, wobei für gleiche oder ähnliche Teile gleiche Bezugszeichen verwendet werden. Unter Umständen werden gleiche oder ähnliche Teile nicht im Zusammenhang mit jeder Figur erneut erläutert.

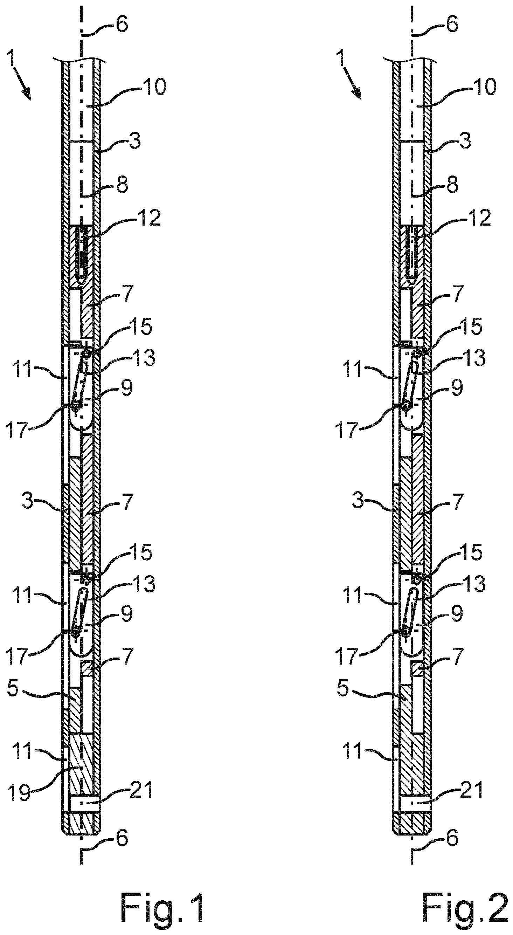

Die in den Fig. 1-7 beschriebene Ausführungsform des erfindungsgemäßen Marknagels 1 umfasst einen im Wesentlichen zylinderförmigen hohlen Rohrkörper 3, in dessen Innerem ein erstes Innenteil 5 sowie ein zweites Innenteil 7 aufgenommen sind. Das erste Innenteil 5 und das zweite Innenteil 7 sind hier im Wesentlichen komplementär als Halbzylinder ausgebildet und erstrecken sich parallel zueinander axial, d. h. in der Längsrichtung des Marknagels 1. Der Marknagel 1 weist an seinem unteren Ende, das in Fig. 1 im unteren Abschnitt angeordnet ist, ein Endstück 19 auf, das zur Fixierung des Marknagels 1 an einem (in Fig. 1 nicht dargestellten) Röhrenknochen dient. Das Endstück 19 weist ein Verriegelungsmittel auf, welches an dem Endabschnitt eines Röhrenknochens befestigbar ist, beispielsweise mit einem Verriegelungsbolzen, der durch entsprechende Öffnungen im Rohrkörper 3 und durch eine Durchgangsöffnung 21 im Endstück 19 geführt ist. Als Verriegelungsbolzen können aus dem Stand der Technik bekannte Bolzen zur Fixierung von Marknägeln in Knochen verwendet werden.

Der Querschnitt des Marknagels 1 und damit des Grundkörpers 3 ist in der hier dargestellten, bevorzugten Ausführungsform kreisförmig. Damit können das erste Innenteil 5 und das zweite Innenteil 7 entsprechend als komplementäre Halbzylinder ausgebildet sein. Auch das untere Endstück 19 weist im Wesentlichen eine Zylinderform auf. Es sei angemerkt, dass der Marknagel 1 auch einen polygonalen Querschnitt aufweisen kann, beispielsweise einen dreieckigen, rechteckigen quadratischen, fünfeckigen, sechseckigen oder achteckigen Querschnitt. Auch ein elliptischer Querschnitt ist möglich. Die Innenteile 5, 7 können dann dazu passend auch mit entsprechendem polygonalen Querschnitt ausgebildet sein.

Im oberen Abschnitt des Marknagels 1 sind ein Getriebe 8 und daran angrenzend ein Motor 10 angeordnet, die zusammen einen Antrieb bilden. Von dem Getriebe 8 angetrieben wird eine Spindel 12, die im Wesentlichen symmetrisch zur Längsachse 6 des Rohrkörpers 3 ausgerichtet ist. Die Spindel 12 umfasst in der hier dargestellten Ausführungsform ein Außengewinde, das in ein Innengewinde im zweiten Innenteil 7 eingreift, so dass eine Drehung der Spindel eine Bewegung des zweiten Innenteils 7 in Axialrichtung bewirkt. Es ist auch denkbar, dass die Funktionsweise umgekehrt erfolgt, d. beispielsweise über eine axial festgelegte, angetriebene Mutter, in der eine Spindel bewegt wird. Die Energieversorgung und Steuerung des Antriebs kann über eine elektrische Verbindung erfolgen, die in den Figuren nicht dargestellt ist. Dabei kann die elektrische Verbindung mit einem Empfänger verbunden sein, der subkutan angeordnet ist, um eine kontaktlose Steuerung und Energieversorgung des Marknagels 1 über eine Steuereinrichtung zu ermöglichen, die sich außerhalb des Körpers des Patienten befindet. Die Energiequelle für den Antrieb kann vorzugsweise außerhalb des Körpers des Patienten angeordnet sein, in weiteren Ausführungsformen auch innerhalb des Marknagels.

Wie bereits oben erwähnt kann der erfindungsgemäßen Marknagel 1 auch andere Antriebe als den hier dargestellten elektromechanischen Antrieb mit Motor 10 und Getriebe 8 aufweisen.

Grundsätzlich beziehen sich in dieser Beschreibung die Begriffe "radial" und "axial" auf die Längsachse 6 des Rohrkörpers 3.

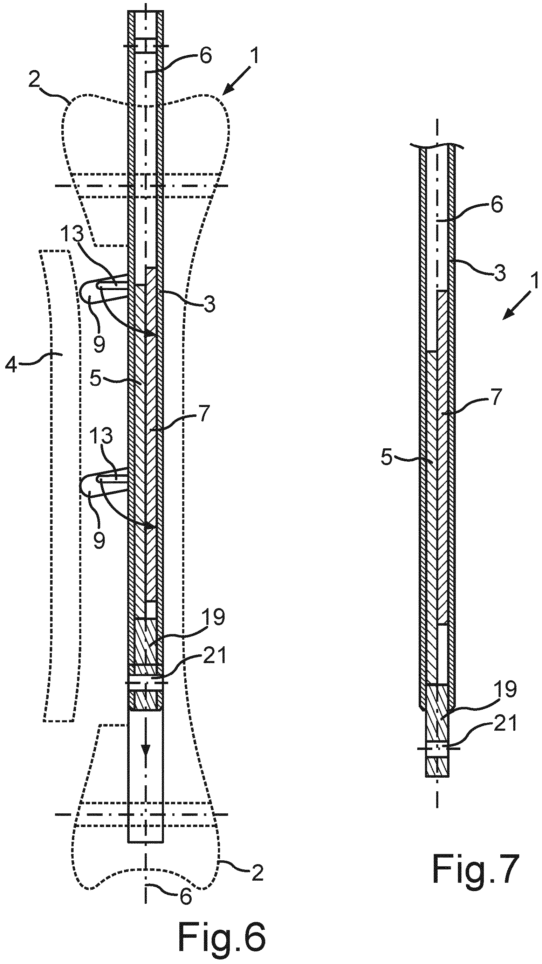

Im Mittelteil weist der Marknagel 1 der hier dargestellten Ausführungsform zwei Längsausnehmungen 11 auf, die im Wesentlichen parallel zur Längsachse 6 ausgerichtet sind und eine Breite von vorzugsweise zwischen 2 mm und 8 mm, noch mehr bevorzugt zwischen 4 mm und 6 mm aufweisen. Auch im unteren Abschnitt weist der Rohrkörper 3 eine Längsausnehmung 11 auf, die im Wesentlichen zur Verriegelung und späteren Entfernung des Marknagels 1 aus dem Röhrenknochen dient, wie weiter unten unter Bezugnahme auf die Fig. 6 und 7 erläutert ist.

Ebenfalls im Mittelbereich auf Höhe der Längsausnehmungen bzw. Langlöcher 11 weist der Marknagel 1 im Inneren in der hier dargestellten Ausführungsform zwei Kipphebel 9 auf, die bei bestimmungsgemäßer Verwendung den Hebelmechanismus bilden, der für die transversale Distraktion verantwortlich ist. Mit anderen Worten, die Kipphebel 9 drehen sich um eine Drehachse derart aus der in Fig. 1 abgebildeten axialen Lage heraus, dass eine radiale Ausdehnungskomponente für die transversale Distraktion sorgt, weil der Kipphebel 9 sich aus der Längsausnehmung 11 heraus bewegt. Der bzw. jeder Kipphebel 9 weist dazu an der einen Seite (in Fig. 1 oben) eine quer zur Längsachse 6 ausgerichtete Bohrung als Drehachse auf, durch die ein Drehbolzen 15 geführt ist. Der Drehbolzen 15 wiederum ist fest am Kipphebel 9 angeordnet und schwenkbar mit dem zweiten Innenteil 7 verbunden. In einer weiteren Ausführungsform ist der Durchmesser des Drehbolzens 15 etwas kleiner als der einer Bohrung durch den Kipphebel 9.

Ein Langloch 13 im Kipphebel 9 ist leicht schräg zu der Längsachse 6 ausgerichtet ausgebildet, wobei quer zur Längsausdehnung des Langlochs 13 ein Führungselement oder Führungsbolzen 17 durch das Langloch 13 geführt ist. Der Führungsbolzen 17 ist fest mit dem ersten Innenteil 5 verbunden.

Der Hebelmechanismus mit dem Kipphebel 9 in der hier dargestellten Ausführungsform wird durch die relative Verschiebung des ersten Innenteils 5 bezogen auf das zweite Innenteil 7 betätigt. Man erkennt im oberen, mittleren Bereich von Fig. 1, dass ein Hohlraum derart zwischen dem ersten Innenteil 5 und dem zweiten Innenteil 7 ausgebildet ist, dass dieser sich bei axialer Bewegung des zweiten Innenteils 7 nach unten, ausgelöst durch die Betätigung des Motors 10, entsprechend verkleinert. Das zweite Innenteil 7 wird also an das erste Innenteil 5 herangeschoben. Weil der Drehbolzen 15 den Kipphebel 9 am Fußpunkt am zweiten Innenteil 7 festhält und der Kipphebel 9 durch die Zwangsführung des Führungsbolzens 17 im Langloch 13 gehalten ist, bewegt sich das in Fig. 1 untere Ende des Kipphebels 9 aus der Längsausnehmung 11 heraus. Diese Kippbewegung bzw. Schwenkbewegung um den Drehbolzen 15 herum ist durch die Länge des Langlochs 13 und den Abstand des zweiten Innenteils 7 bezüglich des ersten Innenteils 5 begrenzt. Die Länge des Langlochs 13 und besagte Abstand sind derart aufeinander abgestimmt, dass ein maximales Schwenken von im Wesentlichen 90° des Kipphebels 9 bezogen auf die Längsachse 6 erreicht wird. Es versteht sich, dass bei der dargestellten Ausführungsform die Abmessungen und Abstände von den beiden Kipphebeln 9 und den Innenteilen 5, 7 gleich sind, so dass sich eine synchrone bzw. abgestimmte Bewegung ergibt. In anderen Ausführungsformen können die Längen der Kipphebel 9 auch unterschiedlich sein. Des Weiteren ist es ebenfalls möglich, dass die Schwenkachsen der Dreh- bzw. Führungsbolzen pro Kipphebel 9 leicht voneinander abweichen. Damit lässt sich eine leicht unterschiedliche Kipprichtung verschiedener Kipphebel 9 erzielen.

Durch die stufenlose Verstellung des Antriebs mit Motor 10, Getriebe 8 und Spindel 12 in beide Richtungen ist eine sehr feine Einstellung der radialen Ausdehnung d. h. der transversalen Distraktion durch den Kipphebel 9 möglich. Damit kann ein Patient selbst den Umfang der transversalen Distraktion durch eine entsprechende Steuerung des Motors 10 von außen einstellen, ohne dass es dazu des Eingriffs durch medizinisches Personal oder gar einen Operateur bedarf.

Fig. 2 zeigt im Wesentlichen denselben Marknagel 1 aus Fig. 1 in einer alternativen Ausführungsform, wobei sich lediglich das untere Ende wie folgt unterscheidet. Anstatt eines separaten Endstücks 19 wie in der in Fig. 1 dargestellten Ausführungsform ist in Fig. 2 das Endstück integral mit dem ersten Innenteil 5 ausgebildet. Dies wiederum bedeutet, dass das untere Ende des Marknagels 1 mit dem entsprechenden Verriegelungsmittel durch das erste Innenteil 5 am Röhrenknochen befestigt wird.

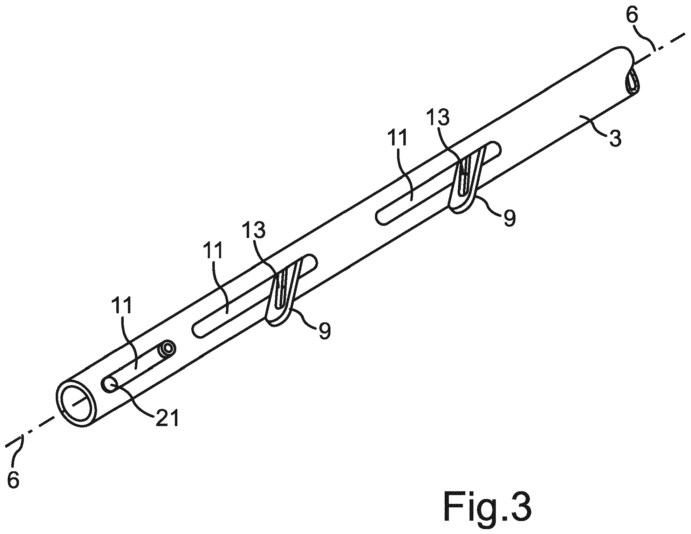

Fig. 3 zeigt in perspektivischer Darstellung die bevorzugte Ausführungsform des erfindungsgemäßen Marknagels 1 in einer zweiten Stellung, in der die Kipphebel 9 ausgefahren sind. Deutlich ist zu sehen, dass der Betrag des radialen Hervorspringens der Kipphebel 9 erheblich größer ist als der Durchmesser des Marknagels 1. Durch geeignete Wahl der Abmessungen der Längsausnehmungen 11 und der Kipphebel 9 kann dieser Betrag optimiert werden. Darüber hinaus erkennt man in Fig. 3, dass der bzw. die Kipphebel 9 nicht vollständig ausgeklappt bzw. herausgefahren sein müssen. Zwischen dem Winkel 0°, also dem eingeklappten Zustand, und dem Winkel 90°, dem vollständig ausgeklappten Zustand, ist im Prinzip jeder Einstellwinkel möglich. Dadurch ergibt sich eine sehr flexible Verwendung des Marknagels zur transversalen Distraktion.

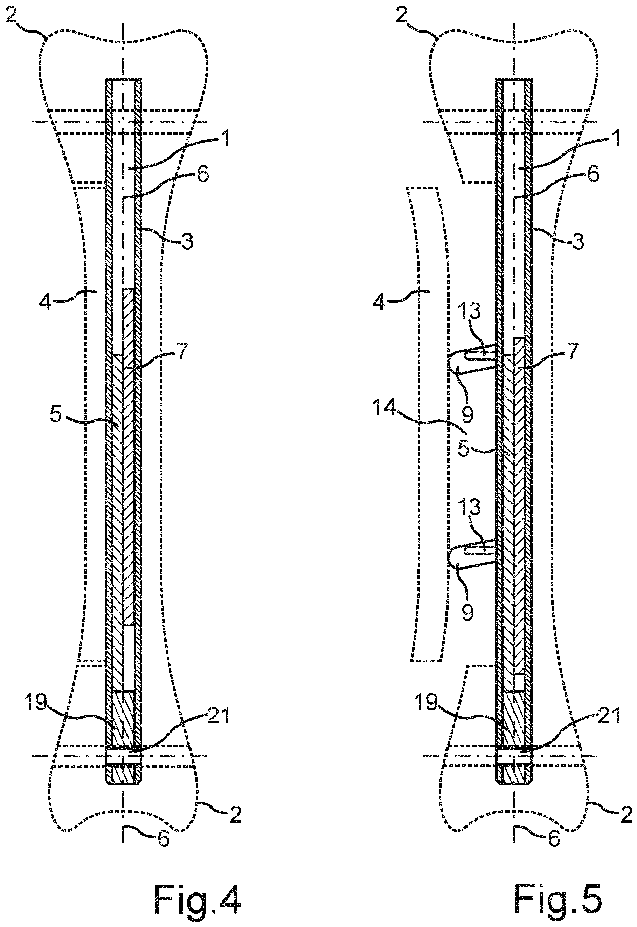

Die Fig. 4 und 5 zeigen eine schematische Querschnittansicht des Marknagels 1 aus Fig. 1 in der ersten bzw. zweiten Stellung in einem Röhrenknochen 2, hier ein Unterschenkelknochen, eingesetzt. Der Röhrenknochen 2 ist hier strichliert dargestellt und ist ausdrücklich nicht Teil der vorliegenden Erfindung. Im unteren sowie im oberen Abschnitt des Röhrenknochens 2 wird der Marknagel 1 über entsprechende Verriegelungsmittel durch Durchgangsöffnungen 21 verdrehsicher und axial fixiert. Diese Art der Fixierung mittels minimalinvasivem Eingriff ist im Stand der Technik bekannt. Zur Vorbereitung der transversalen Distraktion wird ein länglicher Abschnitt 4 im Mittelbereich des Röhrenknochens 2 herausgelöst, d. h. beispielsweise an seinem Umfang mit einer feinen Knochensäge ausgesägt. Der Marknagel 1 liegt über die gesamte Länge des länglichen Abschnitts 4 des Röhrenknochens 2 an diesem in der Stellung der Fig. 4 an. Dies stellt die Ausgangsposition vor der transversalen Distraktion dar, d.h. der Antrieb innerhalb des Marknagels 1 ist noch nicht betätigt worden. Das erste Innenteil 5 ist bezüglich des zweiten Innenteils 7 noch nicht verschoben worden, sondern es besteht dazwischen noch der ursprüngliche axiale Versatz.

Fig. 5 zeigt den Zustand nach Betätigung des Antriebs und damit nach bzw. während der transversalen Distraktion. Das erste Innenteil 5 und das zweiten Innenteil 7 sind durch den (in den Fig. 4 und 5 nicht dargestellten) Antrieb näher ineinander verschoben, wobei sich die Kipphebel 9 aus ihrer in Axialrichtung ausgerichteten Ausgangslage (Fig. 4) in die ausgeklappte bzw. ausgefahrene Distraktionsstellung begeben haben. In der hier dargestellten Ausführungsform haben sich die Kipphebel 9 jeweils um denselben Betrag radial nach außen bewegt durch die Längsausnehmungen 11. Dabei sind sie seitlich an den länglichen Abschnitt 4 des Röhrenknochens 2 angestoßen und haben diesen um einen Distraktionsabstand transversal nach außen bewegt, so dass sich ein Distraktionsraum 14 zwischen der Außenfläche des Rohrkörpers 3 und der Innenfläche des länglichen Abschnitts 4 ergibt. An dieser Stelle sei angemerkt, dass es sich bei der hier gewählten Darstellung um eine schematische handelt.

In Fig. 6 ist eine dritte Stellung des Marknagels 1 der Ausführungsform aus Fig. 1 im Röhrenknochen 2 gezeigt, die eine besondere ist: der Marknagel 1 wird in dieser Stellung aus dem Röhrenknochen 2 entfernt, und zwar axial im Wesentlichen entlang der Längsachse 6. Bevor der Marknagel 1 entfernt werden kann, sind die entsprechenden Verriegelungsmittel am oberen bzw. unteren Ende des Röhrenknochens zu lösen und zu entfernen, damit keine axiale Fixierung mehr vorhanden ist. Wird nun der Marknagel 1 weiter nach oben gezogen, z.B. durch einen Operateur, so sind zunächst die Kipphebel 9 noch ausgefahren, d. h. in ihrer Distraktionsstellung. Sobald der obere Kipphebel 9 jedoch an dem oberen Abschnitt des Röhrenknochens 2 anliegt, also am Ende des länglichen Abschnitts 4, bewirkt eine weitere Bewegung des Marknagels 1 nach oben, dass der Kipphebel 9 einklappt. Dies ist aufgrund der Führung im Langloch 13 möglich. Gleichzeitig bewirkt die Zwangsführung im Langloch 13 auch ein Auseinanderbewegen des ersten Innenteils 5 relativ zum zweiten Innenteil 7, weil der Kipphebel 9 an seinem Fuß über den Drehbolzen 15 am zweiten Innenteil 7 festgelegt ist. Folglich weicht das erste Innenteil 5 relativ zum zweiten Innenteil 7 und auch zum Rohrkörper 3 nach unten aus. Dies wiederum bewirkt eine Verschiebung des Endstücks 19 innerhalb des Rohrkörpers 3 durch das erste Innenteil 5.

Damit ist es möglich, dass beide Kipphebel 9 sich vollständig in den Rohrkörper 3 zurückbewegen und einklappen. Insbesondere wird das Endstück 19 aus dem Rohrkörper 3 nach unten herausgedrückt. Dies kann dadurch erfolgen, dass nach dem Lösen des Verriegelungsmittels durch die Durchgangsöffnung 21 im Endstück 19 keine axiale Fixierung am Knochen mehr besteht und durch das nach oben Ziehen des gesamten Marknagels 1 das Endstück 19 in den frei gewordenen Raum im Röhrenknochen 2 ausweichen kann. Für die medizinische Anwendung bzw. für den Patienten bedeutet dies, dass auch ohne Betätigung des Antriebs oder bei einem Ausfall bzw. einer Störung des Antriebs eine rückstandsfreie, im Wesentlichen problemlose Entfernung der Marknagels 1 bei minimalem operativen Eingriff erfolgen kann. Fig. 7 zeigt schematisch den entfernten Marknagel 1, bei dem die Kipphebel 9 eingeklappt sind und das Endstück 19 unten aus dem Rohrkörper 3 herausragt.

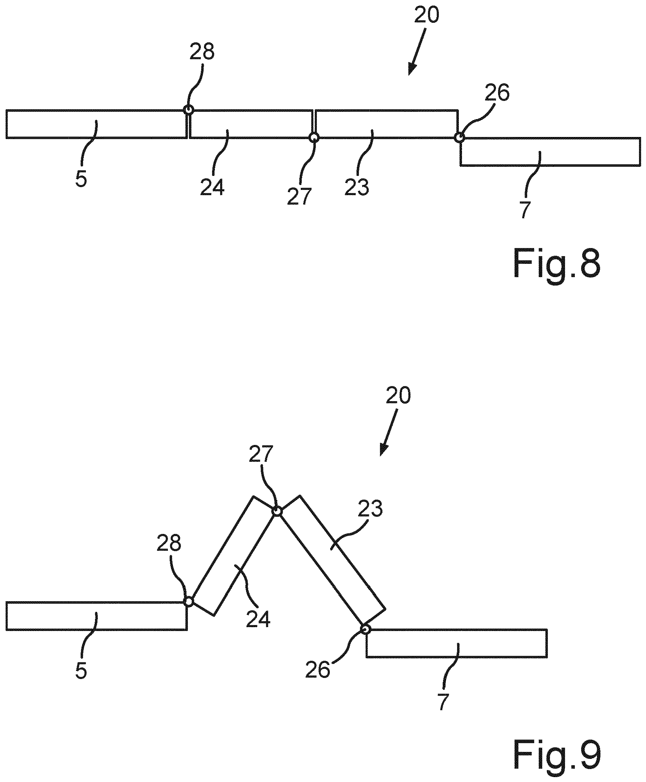

In den Fig. 8 und 9 ist eine alternative Ausführungsform für den Hebelmechanismus des erfindungsgemäßen Marknagels 1 schematisch dargestellt. Fig. 8 zeigt den Hebelmechanismus 20 in eingefahrener Stellung, d. h. wie er innerhalb des Rohrkörpers 3 (nicht dargestellt) axial ausgerichtet ist. An dem zweiten Innenteil 7 ist über eine erste Schwenkachse 26 ein erstes Hebelelement 23 angelenkt, wobei die erste Schwenkachse 26 quer zur Längsachse 6 des Rohrkörpers 3 ausgerichtet ist. Auf der vom zweiten Innenteil 7 distalen Seite des ersten Hebelelements 23 ist über eine zweite Schwenkachse 27 ein zweites Hebelelement 24 angelenkt, wobei die zweite Schwenkachse 27 parallel zur ersten Schwenkachse 26 ausgerichtet ist. Das zweite Hebelelement 24 ist wiederum über eine dritte Schwenkachse 28 gelenkig mit dem ersten Innenteil 5 verbunden, wobei die dritte Schwenkachse 28 ebenfalls parallel zu der ersten Schwenkachse 26 ausgerichtet ist. Der in Fig. 8 und 9 dargestellte Hebelmechanismus ist hier eine Gliederkette oder Schubkette. Derartige Mechanismen lassen sich auch mit segmentierten Bändern realisieren, die auch auf Zug und nicht nur mit Schub funktionieren. Durch die relative Verschiebung der beiden Innenteile lässt sich neben dem in den Figuren dargestellten Schubprinzip auch eine Funktion auf Zug verwirklichen.

Bewegt nun der Antrieb des Marknagels 1 wie beispielsweise oben beschrieben das zweite Innenteil 7 in Richtung des ersten Innenteils 5, das axial bezüglich des Rohrkörpers 3 festgelegt ist, so bewegen sich das erste Hebelelement für 23 und das zweite Hebelelement 24 wie in Fig. 9 dargestellt aus der axialen Ausrichtung entlang der Längsachse 6 in eine Kipphaltung. Dabei bewegen sich beide Hebelelemente 23, 24 durch die Längsausnehmung 11 des Rohrkörpers 3 und denen dabei den Hebelmechanismus 20 quer zur Längsachse 6 aus, so dass dieser zur transversalen Distraktion beiträgt. Das in den Figuren 8 und 9 dargestellte Prinzip des Hebelmechanismus 20 entspricht einer Kettengliedanordnung, wobei gilt, dass die Anzahl der Hebelelemente nicht auf zwei wie in der Darstellung beschränkt ist. Es ergibt sich eine Vielzahl von möglichen Konstruktionen für einen derartigen Hebelmechanismus 20. Beispielsweise müssen die Hebelelemente nicht notwendigerweise gerade ausgebildet sein. Entscheidend ist, dass sich durch die relative Verschiebung des ersten Innenteils 5 bezüglich des zweiten Innenteils 7 eine Auffaltung der Hebelelemente ergibt, die sich dann aus der Längsausnehmung des Rohrkörpers in axialer Richtung herausbewegen. Darüber hinaus sollte eine Bewegung der Innenteile 5, 7 in entgegengesetzter Richtung ein Einfalten bzw. ein Einfahren des Hebelmechanismus 20 bewirken.

Ähnlich dem in den Fig. 8 und 9 dargestellten Prinzip ist auch ein Kniehebelmechanismus denkbar, bei dem ein Ende eines ersten Hebelelements im mittleren Bereich eines zweiten Hebelelements gelenkig befestigt ist.

### Bezugszeichenliste:

- 1: Marknagel
- 2: Röhrenknochen
- 3: Rohrkörper
- 4: Knochensegment/länglicher Abschnitt
- 5: erstes Innenteil
- 6: Längsachse
- 7: zweites Innenteil
- 8: Getriebe
- 9: Kipphebel
- 10: Motor
- 11: Längsausnehmung
- 12: Spindel
- 13: Langloch
- 14: Distraktionsraum
- 15: Drehbolzen
- 17: Führungselement
- 19: Endstück
- 20: Hebelmechanismus
- 21: Durchgangsöffnung
- 23: erstes Hebelelement
- 24: zweites Hebelelement
- 26: erste Schwenkachse
- 27: zweite Schwenkachse
- 28: dritte Schwenkachse

## Patentansprüche

1. Marknagel (1) zur transversalen Distraktion eines Röhrenknochens (2), wobei der Marknagel (1) umfasst:
- einen sich in einer axialen Richtung des Marknagels (1) erstreckenden, zumindest teilweise hohlen Rohrkörper (3),
- mindestens ein Verriegelungsmittel zum Verriegeln des Rohrkörpers (3) in einem Endabschnitt des Röhrenknochens (2),
- ein erstes Innenteil (5) und ein zweites Innenteil (7), die jeweils innerhalb des Rohrkörpers (3) in axialer Richtung gegeneinander oder miteinander verschiebbar angeordnet sind,
- einen Antrieb innerhalb des Rohrkörpers (3) zum axialen Verschieben des ersten Innenteils (5) relativ zu dem zweiten Innenteil (7),
- mindestens eine Längsausnehmung (11) des Rohrkörpers (3),
- mindestens einen Hebelmechanismus (20), der mindestens ein Hebelelement (9, 23, 24) umfasst, das dazu eingerichtet ist, in radialer Richtung aus dem Rohrkörper (3) ausgefahren und wieder eingefahren zu werden,
- wobei die Verschiebung des ersten Innenteils (5) bezüglich des zweiten Innenteils (7) ein Herausbewegen bzw. Ausfahren des mindestens einen Hebelelements (9) aus der mindestens einen seitlichen Längsausnehmung (11) des Rohrkörpers (3) oder ein Hineinbewegen bzw. Einfahren des mindestens einen Hebelelements (9) in die mindestens eine seitliche Längsausnehmung (11) des Rohrkörpers (3) bewirkt.

2. Marknagel (1) nach Anspruch 1, **dadurch gekennzeichnet, dass** das mindestens eine Hebelelement (9, 23, 24) in einer eingefahrenen Stellung axial mit der Längsachse (6) des Marknagels (1) ausgerichtet ist und in einer ausgefahrenen Stellung um mindestens 30°, bevorzugt um mindestens 45°, noch bevorzugter um mindestens 60° und am meisten bevorzugt um mindestens 80° bezüglich der Längsachse (6) des Marknagels (1) ausgerichtet ist.

3. Marknagel (1) nach einem der vorhergehenden Ansprüche, **dadurch gekennzeichnet, dass** der Antrieb axial am Rohrkörper (3) abgestützt ist.

4. Marknagel (1) nach einem der vorhergehenden Ansprüche, **dadurch gekennzeichnet, dass** das mindestens eine Hebelelement (9, 23, 24) dazu eingerichtet ist, bei Bewegung des Marknagels (1) in Längsrichtung ohne Betätigung des Antriebs eingefahren zu werden.

5. Marknagel (1) nach einem der vorhergehenden Ansprüche, **dadurch gekennzeichnet, dass** das zweite Innenteil (7) integral mit dem Rohrkörper (3) ausgebildet ist.

6. Marknagel (1) nach einem der vorhergehenden Ansprüche, **dadurch gekennzeichnet, dass** der Antrieb als ein elektrischer, magnetischer, elektro-magnetischer, hydraulischer, formgedächtnisbasierter, piezoelektrischer oder pneumatischer Antrieb ausgebildet ist.

7. Marknagel (1) nach einem der vorhergehenden Ansprüche, **dadurch gekennzeichnet, dass** der Antrieb einen Motor (10), ein Getriebe (8) und vorzugsweise ein Spindelelement (12) aufweist.

8. Marknagel (1) nach einem der vorhergehenden Ansprüche, **dadurch gekennzeichnet, dass** das erste Innenteil (5) und das zweite Innenteil (7) eine Halbzylinderform aufweisen.

9. Marknagel (1) nach einem der vorhergehenden Ansprüche, **dadurch gekennzeichnet, dass** das mindestens eine Hebelelement als Kipphebel (9) ausgebildet ist, der ein Langloch (13) und eine Gelenkeinrichtung aufweist, wobei die Gelenkeinrichtung drehbar an und axial festgelegt mit dem ersten Innenteil (5) verbunden ist und wobei das zweite Innenteil (7) ein Führungselement (17) aufweist, das in dem Langloch (13) des Kipphebels (9) geführt ist.

10. Marknagel (1) nach einem der Ansprüche 1 bis 8, **dadurch gekennzeichnet, dass** das mindestens eine Hebelelement als eine Mehrzahl von gelenkig miteinander verbundenen Kettengliedern (23, 24) ausgebildet ist, wobei ein Kettenglied der Mehrzahl von Kettengliedern axial an das erste Innenteil (5) angrenzt und ein anderes der Mehrzahl von Kettengliedern an das zweite Innenteil (7) angrenzt, wobei die Verschiebung des ersten Innenteils (5) relativ zu dem zweiten Innenteil (7) ein Auffalten und Hervortreten von mindestens zwei Kettengliedern durch die seitliche Längsausnehmung (11) des Rohrkörpers (3) bewirkt.

## Claims

1. An intramedullary nail (1) for transverse distraction of a tubular bone (2), the intramedullary nail (1) comprising:
- an at least partially hollow tube body (3) extending in an axial direction of the intramedullary nail (1),
- at least one locking means for locking the tube body (3) in an end portion of the tubular bone (2),
- a first inner part (5) and a second inner part (7), which are each arranged within the tube body (3) so as to be displaceable in the axial direction counter to each other or with each other,
- a drive within the tube body (3) for axially displacing of the first inner part (5) relative to the second inner part (7),
- at least one longitudinal recess (11) in the tube body (3),
- at least one lever mechanism (20) which comprises at least one lever element (9, 23, 24) which is configured to be extended out of, and to be retracted again into, the tube body (3) in a radial direction,
- wherein the displacement of the first inner part (5) with respect to the second inner part (7) causes the at least one lever element (9) to be moved or extended out of the at least one lateral longitudinal recess (11) of the tube body (3) or causes the at least one lever element (9) to be moved or retracted into the at least one lateral longitudinal recess (11) of the tube body (3).

2. The intramedullary nail (1) according to claim 1, **characterized in that** the at least one lever element (9, 23, 24) in a retracted position is aligned axially with the longitudinal axis (6) of the intramedullary nail (1) and in an extended position is aligned by at least 30°, preferably by at least 45°, even more preferably by at least 60° and most preferably by at least 80° with respect to the longitudinal axis (6) of the intramedullary nail (1).

3. The intramedullary nail (1) according to any one of the preceding claims, **characterized in that** the drive is supported axially on the tube body (3).

4. The intramedullary nail (1) according to any one of the preceding claims, **characterized in that** the at least one lever element (9, 23, 24) is configured to be retracted in the longitudinal direction without actuation of the drive during movement of the intramedullary nail (1).

5. The intramedullary nail (1) according to any one of the preceding claims, **characterized in that** the second inner part (7) is integrally formed with the tube body (3).

6. The intramedullary nail (1) according to any one of the preceding claims, **characterized in that** the drive is configured as an electrical, magnetic, electro-magnetic, hydraulic, shape memory based, piezoelectric or pneumatic drive.

7. The intramedullary nail (1) according to any one of the preceding claims, **characterized in that** the drive comprises a motor (10), a transmission (8) and preferably a spindle element (12).

8. The intramedullary nail (1) according to any one of the preceding claims, **characterized in that** the first inner part (5) and the second inner part (7) comprise a half-cylinder shape.

9. The intramedullary nail (1) according to any one of the preceding claims, **characterized in that** the at least one lever element is formed as a rocker arm (9), which has an elongate hole (13) and a joint device, wherein the joint device is rotatably connected on and axially fixed to the first inner part (5), and wherein the second inner part (7) has a guide element (17) which is guided in the elongate hole (13) of the rocker arm (9).

10. The intramedullary nail (1) according to any one of claims 1 to 8, **characterized in that** the at least one lever element is formed as a plurality of chain links (23, 24) connected to one another in an articulated manner, wherein one chain link of the plurality of chain links is axially adjacent to the first inner part (5) and another of the plurality of chain links adjoins the second inner part (7), wherein the displacement of the first inner part (5) relative to the second inner part (7) causes folding and protrusion of at least two chain links through the lateral longitudinal recess (11) of the tube body (3).

## Revendications

1. Clou centromédullaire (1) pour extension transversale d'un os long (2), dans lequel le clou centromédullaire (1) comprend :
- un corps tubulaire (3) au moins partiellement creux s'étendant dans une direction axiale du clou centromédullaire (1),
- au moins un moyen de verrouillage destiné à verrouiller le corps tubulaire (3) dans une section terminale de l'os long (2),
- une première partie intérieure (5) et une deuxième partie intérieure (7), lesquelles sont respectivement disposées à l'intérieur du corps tubulaire (3) de façon à pouvoir se déplacer l'une contre l'autre ou l'une par rapport à l'autre dans la direction axiale,
- un entraînement à l'intérieur du corps tubulaire (3) destiné au déplacement axial de la première partie intérieure (5) par rapport à la deuxième partie intérieure (7),
- au moins un évidement longitudinal (11) du corps tubulaire (3),
- au moins un mécanisme à levier (20) comprenant au moins un élément de levier (9, 23, 24), lequel est conçu pour être déployé dans la direction radiale hors du corps tubulaire (3) et être à nouveau escamoté,
- dans lequel le déplacement de la première partie intérieure (5) par rapport à la deuxième partie intérieure (7) opère un mouvement d'extraction ou de déploiement de l'au moins un élément de levier (9) hors de l'au moins un évidement longitudinal latéral (11) du corps tubulaire (3) ou un mouvement de rétraction ou d'escamotage de l'au moins un élément de levier (9) dans l'au moins un évidement longitudinal latéral (11) du corps tubulaire (3).

2. Clou centromédullaire (1) selon la revendication 1, **caractérisé en ce que** l'au moins un élément de levier (9, 23, 24) est, dans une position escamotée, orienté axialement dans l'axe longitudinal (6) du clou centromédullaire (1) et est, dans une position déployée, orienté à au moins 30°, de préférence à au moins 45°, plus préférablement à au moins 60° et le plus préférablement à au moins 80° par rapport à l'axe longitudinal (6) du clou centromédullaire (1).

3. Clou centromédullaire (1) selon l'une des revendications précédentes, **caractérisé en ce que** l'entraînement s'appuie axialement sur le corps tubulaire (3).

4. Clou centromédullaire (1) selon l'une des revendications précédentes, **caractérisé en ce que** l'au moins un élément de levier (9, 23, 24) est orienté de façon à se rétracter sans actionnement de l'entraînement en cas de mouvement du clou centromédullaire (1) dans la direction longitudinale.

5. Clou centromédullaire (1) selon l'une des revendications précédentes, **caractérisé en ce que** la deuxième partie intérieure (7) est réalisée de manière intégrée au corps tubulaire (3).

6. Clou centromédullaire (1) selon l'une des revendications précédentes, **caractérisé en ce que** l'entraînement est réalisé comme entraînement électrique, magnétique, électromagnétique, hydraulique, basé sur la mémoire de forme, piézoélectrique ou pneumatique.

7. Clou centromédullaire (1) selon l'une des revendications précédentes, **caractérisé en ce que** l'entraînement comporte un moteur (10), une transmission (8) et de préférence un élément de broche (12).

8. Clou centromédullaire (1) selon l'une des revendications précédentes, **caractérisé en ce que** la première partie intérieure (5) et la deuxième partie intérieure (7) comportent une forme semi-cylindrique.

9. Clou centromédullaire (1) selon l'une des revendications précédentes, **caractérisé en ce que** l'au moins un élément de levier est réalisé comme levier basculant (9), lequel comporte un trou oblong (13) et un dispositif d'articulation, dans lequel le dispositif d'articulation est relié à la première partie intérieure (5) de manière rotative et axialement fixée et dans lequel la deuxième partie intérieure (7) comporte un élément de guidage (17), lequel est guidé dans le trou oblong (13) du levier basculant (9).

10. Clou centromédullaire (1) selon l'une des revendications 1 à 8, **caractérisé en ce que** l'au moins un élément de levier est réalisé comme une pluralité de maillons de chaîne (23, 24) reliés l'un à l'autre de manière articulée, dans lequel un maillon de chaîne de la pluralité de maillons de chaîne jouxte axialement la première partie intérieure (5) et un autre de la pluralité de maillons de chaîne jouxte la deuxième partie intérieure (7), dans lequel le déplacement de la première partie intérieure (5) par rapport à la deuxième partie intérieure (7) opère un dépliage et une saillie d'au moins deux maillons de chaîne à travers l'évidement longitudinal latéral (11) du corps tubulaire (3).
